# EUROPEAN PATENT SPECIFICATION

(11) **EP 2 749 235 B1**
(45) Date of publication and mention of the grant of the patent: **09.08.2017**
(21) Application number: 12199797.7
(22) Date of filing: 31.12.2012
(51) Int. Cl.: A61B 17/17, A61B 17/00, A61B 17/56

(54) **Patient specific operative guide for use in spinal surgery**
Patientenspezifische Operationsführung zur Verwendung in der Wirbelsäulenchirurgie
Guide de fonctionnement spécifique au patient destiné à être utilisé en chirurgie vertébrale

(43) Date of publication of application: 02.07.2014
(73) Proprietor: Medacta International S.A., 6874 Castel San Pietro (TI) (CH)
(72) Inventor: Fiechter, Meinrad, 6874 Castel San Pietro (CH); Lipari, Alberto, 6874 Castel San Pietro (CH); Siccardi, Francesco, 6874 Castel San Pietro (CH)
(74) Representative: Inchingalo, Simona

(56) References cited:
- EP-A1- 2 502 582
- TW-A- 201 238 556
- US-A1- 2011 319 745
- US-A1- 2012 245 647

## Description

### Field of the invention

The present invention relates to the broad technical field of orthopedic surgery. More specifically, the invention relates to a patient-specific operative guide to be employed in spinal surgery.

### Related art

The groundbreaking works of Radermacher et al. in the early nineties have paved the way for the now widespread use of patient specific guides in orthopedic surgery.

Patient specific guides are disposable templates, which are individually designed to match the bone anatomy derived from CT-scans of a given patient. Surgical operations like drills and cuts can be pre-operatively planned by computer-aided technologies, and the resulting patient specific guides will later allow the surgeon to accurately replicate the planned operations on the patient's body. Examples of patient specific guides are shown in documents EP 2502582, TW 201238556 and US 2011/319745. Patient specific guides have been employed in several fields of orthopedic surgery, including spinal surgery.

In this field, patient specific guides are mainly employed to help the surgeon during pedicle screw insertion, so that the screw can be inserted according to a pre-planned optimal screw axis.

However, patient specific guides may be used in spinal surgery for other purposes; for instance as cutting guides during PSO (Pedicle substraction osteotomies), laminotomy or facectomies.

However, the introduction of patient specific guides in the field of spinal surgery has proven to be challenging.

Indeed, the guides have to be designed in such a way that they couple with the patient's vertebrae in a stable and well-defined configuration. In order to achieve this goal, it is necessary to have large contact areas between the guide and the bony structure of the patient.

Therefore, before the positioning of the guide, the surgeon is forced to clean a large area of the bone from the surrounding tissue, and in some cases to severe the ligaments. This often proves to be a difficult and time-consuming task, and may lead to complication and lengthening of the patient recovery.

Moreover, the remaining tissue that the surgeon is unable to remove may lead to slippery and deviation of the guide, eventually resulting in an incorrect or suboptimal positioning of the pedicle screws or bone resections.

In view of the foregoing, the technical problem underlying the present invention is to provide a patient specific surgical guide, of the type being used in spinal surgery, which stably and uniquely couples with the vertebrae of a patient without resorting to large contact areas.

### Summary of the invention

The invention is defined by the claims.

The above-mentioned technical problem is solved by a patient specific operative guide for use in spinal surgery, comprising at least one guiding member for guiding a surgical operation on a patient's vertebra; and a plurality of contact members designed to match with a corresponding plurality of contact areas on the patient's vertebra in order to define a unique coupling configuration of the patient specific operative guide on the patient's vertebra, wherein said contact members comprise a main contact member, designed to couple with a main contact area corresponding to the spinous process of the patient's vertebra in said coupling configuration, and at least one pair of auxiliary contact members, designed to abut on auxiliary contact areas, respectively positioned laterally left and laterally right of the spinous process, in said coupling configuration.

The main contact member can define a seat into which the spinous process fits. Said seat may be U-shaped and opening on both the cranial-caudal sides (open profile). Alternatively, the seat may open only on one of the cranial-caudal sides, the opposite side being closed by a top wall so as to partially enclose the spinous process in the coupling configuration (semi-open profile). Alternatively, both cranial-caudal sides of the seat may be closed respectively by a top wall and by a bottom wall, so as to completely enclose the spinous process in the coupling configuration (closed profile).

The above-described closed profile ensures a more stable positioning of the operative guide, but requests the severing of ligaments between spinous process. The open profile is less stable, but less invasive as the ligaments do not need to be severed. The semi-open profile is a trade-off between the opposing requirements of stability and non-invasiveness.

Several contact schemes, employing three or more contact points, can be selected for coupling the operative guide to the patient's vertebra.

The auxiliary contact areas can lie on the two opposite laminae of the patient's vertebra.

Alternatively, particularly in the case of guides specifically designed to match with a lumbar, cervical or thoracic vertebra, the auxiliary contact areas can correspond to the articular processes of the patient's vertebra.

Alternatively, particularly in the case of guides specifically designed to match with a lumbar or thoracic vertebra, the auxiliary contact areas can correspond to the transverse processes of the patient's vertebra.

The contact members can be designed to match with five separate contact areas, i.e. a five-points contact scheme is used for coupling the operative guide to the patient's vertebra. The auxiliary contact members thus comprise: a pair of first auxiliary contact members, matching with first auxiliary contact areas respectively positioned laterally left and laterally right of the spinous process; and a pair of second auxiliary contact members, matching with second auxiliary contact areas respectively positioned laterally left and laterally right of the first auxiliary contact areas.

The above-described five-points contact scheme ensures an optimal stability for the operative guide, without resorting to the large contact areas that would require extensive intra-operative cleaning of the bony structure.

In the case of a guide specifically designed to match with a lumbar vertebra, the first auxiliary contact areas can advantageously correspond to the upper articular processes of the patient's vertebra, the second auxiliary contact areas corresponding to the transverse processes of the patient's vertebra.

Alternatively, particularly in the case of guides specifically designed to match with a lumbar, cervical or thoracic vertebra, the first auxiliary contact areas can advantageously lie on the laminae of the patient's vertebra, the second auxiliary contact areas corresponding to the articular processes of the patient's vertebra.

Alternatively, particularly in the case of guides specifically designed to match with a lumbar or thoracic vertebra, the first auxiliary contact areas can advantageously lie on the laminae of the patient's vertebra, the second auxiliary contact areas corresponding to the transverse processes of the patient's vertebra.

The guiding members can comprise at least a tubular member adapted to be used as a drill guide.

The diameter of said tubular member can be such as to allow the insertion of a pedicle screw, the patient specific guide further comprising an adapter sleeve insertable within the tubular member and featuring a hole for guiding a Kirchner wire, as well as drills, taps, or other instruments that can be used to open/prepare the pedicle/vertebral body.

The guiding members can also comprise at least a slotted member adapted to be used as a resection guide in order to perform e.g. osteostomies (not forming part of the invention).

Further features and advantages of the patient specific operative guide according to the invention shall be made clearer by the description, given herein below, of a number of embodiments described by way of non-limiting example with reference to the accompanying drawings.

### Brief description of the drawings

Figures 1-3 show perspective views of a first embodiment of the patient specific operative guide according to the invention, coupled to a lumbar vertebra;
Figures 4-6 show perspective views of a second embodiment of the patient specific operative guide according to the invention, coupled to a thoracic vertebra;
Figures 7-9 show perspective views of a third embodiment of the patient specific operative guide according to the invention, coupled to a cervical vertebra;
Figure 10 shows an enlarged feature of the patient specific operative guide from figures 1-3;
Figures 11-13 show alternative arrangements for the specific feature depicted in figure 10;
Figures 14-16 show front views of three subsequent steps of a surgical method employing a patient specific guide t according to the present invention;
Figures 17-19 show perspective views of the three method steps depicted in figures 14-16.

### Detailed description

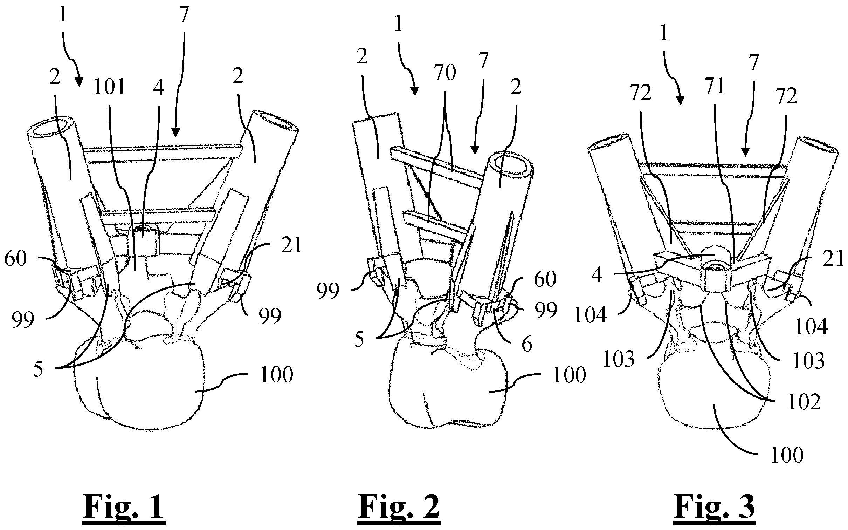

Referring to figures 1-3, a first embodiment of a patient specific operative guide 1 for spinal surgery is illustrated, which is specifically designed for operations on a lumbar vertebra 100.

As may be readily recognized in these figures, the operative guide 1 comprises two tubular guiding members 2 integral with a bearing frame 7.

The two tubular guiding members 2 define the insertion axes for two pedicle screws, which should be inserted in the lumbar vertebra according to a pre-operatively planned angle. Therefore, the tubular guiding members 2 feature an upper opening, wherefrom a surgical tool is inserted, and a lower opening in the vicinity of the patient's vertebra.

The diameter of the tubular guiding members 2 is such as to allow the insertion of a pedicle screw. However, the patient specific operative guide 1 may also comprise an adapter sleeve insertable on the upper opening of the tubular guiding member 2, and featuring a central hole for guiding a Kirchner wire.

An open window 21 is left at the lower opening so that the surgeon can check the entry point of the pedicle screw or Kirchner wire inserted through the tubular guiding members 2.

The bearing frame comprises a V-shaped bridge 71, connecting the two tubular guiding members 2 at the level of the lower openings, and one or more straight transverse bars 70 (two in the present embodiment) connecting together upper sections of the tubular members 2.

The V-shaped bridge 71 has a substantially planar extension, and points toward the caudal direction, so that its vertex is positioned above the spinous process of the lumbar vertebra 100. Reinforcing ribs 72 are provided that connect the arms of the V-shaped bridge 71 to the upper extension of the tubular members 2.

The pre-operatively planning is performed, by means of computer-aided design tools, on a three-dimensional model of the bony structure developed from a three-dimensional image (e.g. CT/MRI scan) of the patient. Therefore, the operative guide 1 is designed in such a way that it uniquely matches the bony structure of the patient.

In particular, to ensure a correct and stable positioning of the operative guide 1, five separate contact members 4, 5, 6 are provided, each of them being designed to match with a corresponding contact area on the patient's vertebra 100.

A main contact member 4 is intended to couple with a main contact area, corresponding to the spinous process 101 of the vertebra 100.

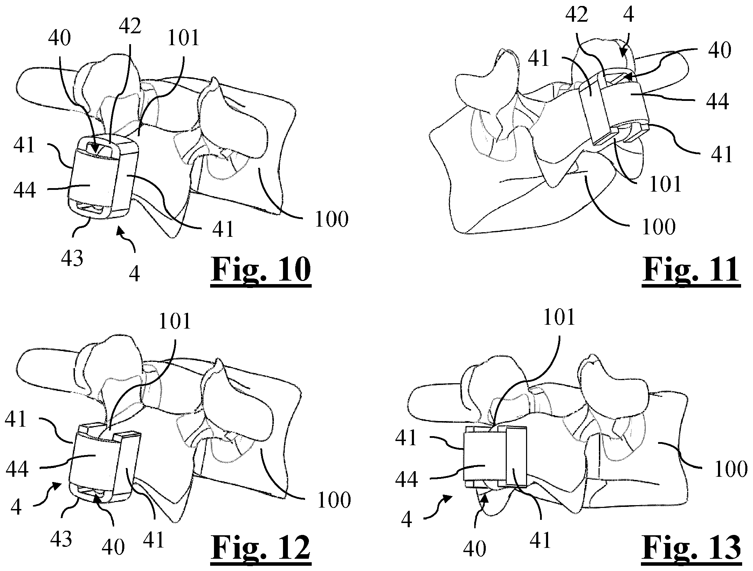

The main contact member 4, which is located at the vertex of the V-shaped bridge 71, is better seen in figure 10 in which the other features of the operative guide have been omitted for the sake of clarity.

As readily acknowledgeable from figure 10, the main contact member 4 defines a seat 40 that extends around the spinous process. The seat 40 is mainly defined by a pair of lateral walls 41 bridged together by an arch-like structure 44. The arms of the V-shaped bridge 71 depart from the external sides of said lateral walls 41.

The seat 40 is closed in the cranial-caudal direction by a top wall 42 and a bottom wall 43, which connect to the lateral walls 41 forming a closed profile surrounding the spinous process 101. The closed profile ensures an excellent stability to the operative guide 1; however the ligaments of the patients need to be severed before placing the guide in the coupling configuration.

Figures 11-13 show alternative embodiments for the main contact member 4, wherein one or both of the top/bottom walls are not provided in order to allow for a less invasive operative technique.

In particular, figure 11 depicts a main contact member 4 without bottom wall (semi-open profile), figure 12 depicts a main contact member 4 without top wall (semi-open profile) and figure 13 depicts a main contact member 4 without both bottom and top walls (open profile).

The contact members 4, 5, 6 further comprise a pair of first auxiliary contact members 5, matching with first auxiliary contact areas, and a pair of second auxiliary contact members 6, matching with second auxiliary contact areas. In the present embodiment, the first auxiliary contact areas correspond to the upper articular processes 103 of the vertebra, while the second auxiliary contact areas correspond to the transverse processes 104.

However, in alternative embodiments, one or both of the contact areas can lie on the laminae 102 of the patient's vertebra 100.

The two first auxiliary contact members 5, as well as the two second auxiliary contact members 6, are symmetrically positioned on the operative guide 1, with respect to a median plane passing through the main contact member 4. However, depending on the patent anatomy a unsymmetrical arrangement is also possible.

The two second auxiliary contact members 6 are laterally placed with respect to the first auxiliary contact members 5; in other words, the first auxiliary contact members 5 are positioned between the main contact member 4 and the second auxiliary contact members 6.

In the present embodiment, each of the first auxiliary contact members 5 comprises a single contact finger, projecting downwards from the lower opening of a respective tubular guiding member 2. The free end of said contact fingers is designed with a concave shape matching with the upper articular process 103 of the patient's vertebra 100. It is noted that the contact finger extends from a cranial/inner portion of the guiding member's lower opening, i.e. from a rim sector which is directed toward the median plane and away from the vertex of the V-shaped bridge 71.

In the present embodiment, each of the second auxiliary contact members 6 is a middle contact finger, projecting downward from an outer lateral plate 60 neighboring the lower opening of a respective tubular guiding member 2. A longitudinal rib connects the outer lateral plate 60 to the main body of the tubular member 2. The middle contact finger 6 is designed to match with the transverse process 104 of the patient's vertebra 100.

Two lateral indicator fingers 99 are provided at the sides of the middle contact finger 6, substantially aligned along a cranial-caudal direction. These lateral indicator fingers 99 are not placed in direct contact with the transverse process 104, but rest separated from it by a small gap (preferably equal to about 1 mm). The lateral indicator fingers 99 are used as indicators to show to the user the approximate position of the guide on the transverse process.

Note that a different number of fingers extending from the outer lateral plate 60 could be envisaged.

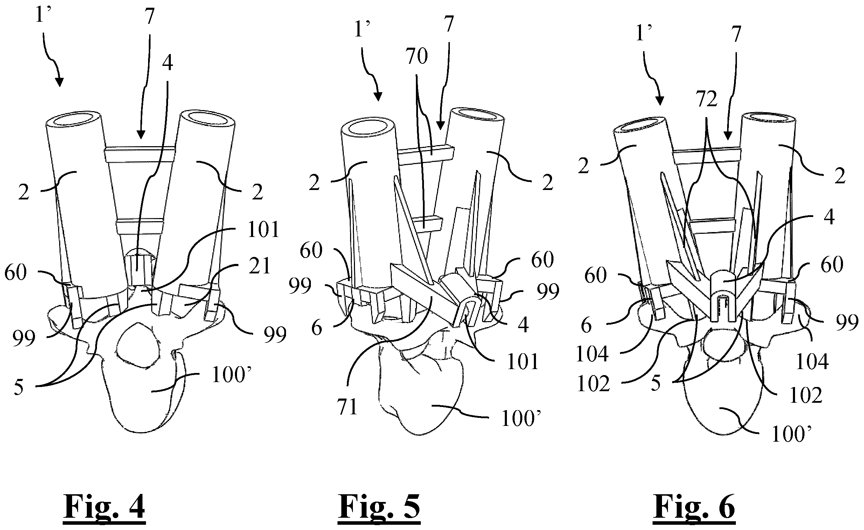

Referring now to figures 4-6, a second embodiment of a patient specific operative guide 1' for spinal surgery is illustrated, which is specifically designed for operations on a thoracic vertebra 100'.

The second embodiment shares most of the features of the first embodiment. Features which are identical or similar in structure or function are indeed identified with the same reference number in the attached drawings. In the following description, only the technical aspects substantially different from those of the first embodiment are explicitly addressed.

The patient specific operative guide 1' according to the second embodiment also comprises two tubular guiding members 2, integral with a bearing frame 7 with transverse bars 70 and a V-shaped bridge 71. Compared to the first embodiment, the V-shaped bridge 71 extends for a longer distance in the caudal direction, so as to reach the spinous process 101 which is farther from the vertebral body in thoracic vertebrae 100'.

In the depicted embodiment, the main contact member 4 has a semi-open profile with no bottom wall; however, different configurations according to layouts of figures 10-13 may be envisaged.

In this second embodiment, the first auxiliary contact areas matching with the first auxiliary contact members 5 lie on the laminae 102 of the vertebra 100'; the second auxiliary contact areas matching with the second auxiliary contact members 6 correspond to the transverse processes 104 of the vertebra 100'.

Each of the first auxiliary contact members 5 comprises a single contact finger extending from a caudal/inner portion of the guiding member's lower opening, i.e. from a rim sector which is directed toward the median plane and toward the vertex of the V-shaped bridge 71.

Each of the second auxiliary members 6 has a middle contact finger aligned with two lateral indicator fingers 99, all of them extending from the outer lateral plate 60 of the corresponding tubular guiding member 2.

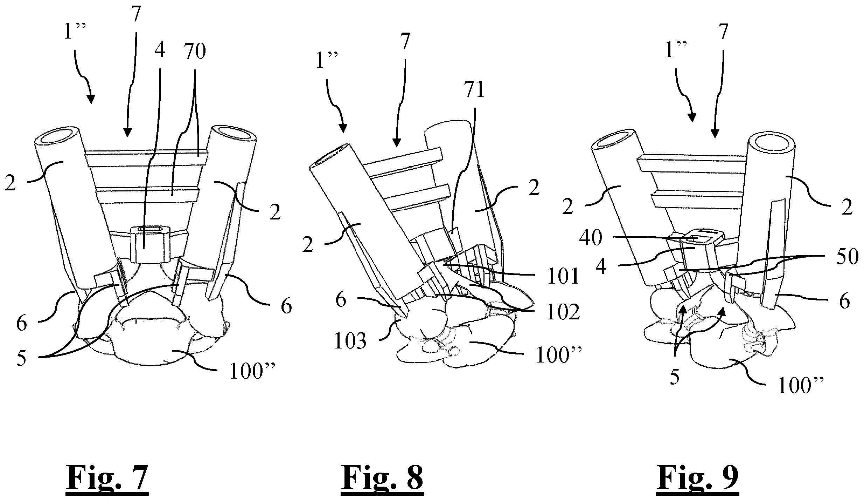

Referring now to figures 7-9, a third embodiment of a patient specific operative guide 1" for spinal surgery is illustrated, which is specifically designed for operations on a cervical vertebra 100".

The third embodiment shares most of the features of the first embodiment. Features which are identical or similar in structure or function are indeed identified with the same reference number in the attached drawings. In the following description, only the technical aspects substantially different from those of the first embodiment are explicitly addressed.

The patient specific operative guide 1" according to the third embodiment also comprises two tubular guiding members 2, integral with a bearing frame 7 with transverse bars 70 and a V-shaped bridge 71. Compared to the first embodiment, the V-shaped bridge 71 extends for a shorter distance in the caudal direction, since the spinous process 101 is closer to the vertebral body in cervical vertebrae 100". Moreover, given the compactness of this embodiment, the V-shaped bridge 71 is not connected to the tubular members 2 by reinforcing ribs.

In the depicted embodiment, the main contact member 4 has a closed profile with top and bottom walls but does not feature an arch-like structure.

In this third embodiment, the first auxiliary contact areas matching with the first auxiliary contact members 5 lie on the laminae 102 of the vertebra 100"; the second auxiliary contact areas matching with the second auxiliary contact members 6 correspond to the articular processes 103 or laminae of the vertebra 100".

Each of the first auxiliary contact members 5 comprises three contact fingers, projecting downward from an inner lateral plate 50 neighboring the lower opening of a respective tubular guiding member 2. A longitudinal rib connects the inner lateral plate 50 to the main body of the tubular member 2. The contact fingers are substantially aligned along a cranial-caudal direction, and are designed to match with the lamina 102 of the patient's vertebra 100. A different number of contact fingers extending from the inner lateral plate 50 could be envisaged.

On the contrary, each of the second auxiliary contact members 6 is now formed by a contact finger, not surrounded by lateral indicator fingers, projecting downwards directly from the lower opening of a respective tubular guiding member 2. Therefore, no outer lateral plate is provided in the present embodiment. The sole contact finger extends from a caudal/outer portion of the guiding member's lower opening, i.e. from a rim sector which is directed away from the median plane and toward the vertex of the V-shaped bridge 71.

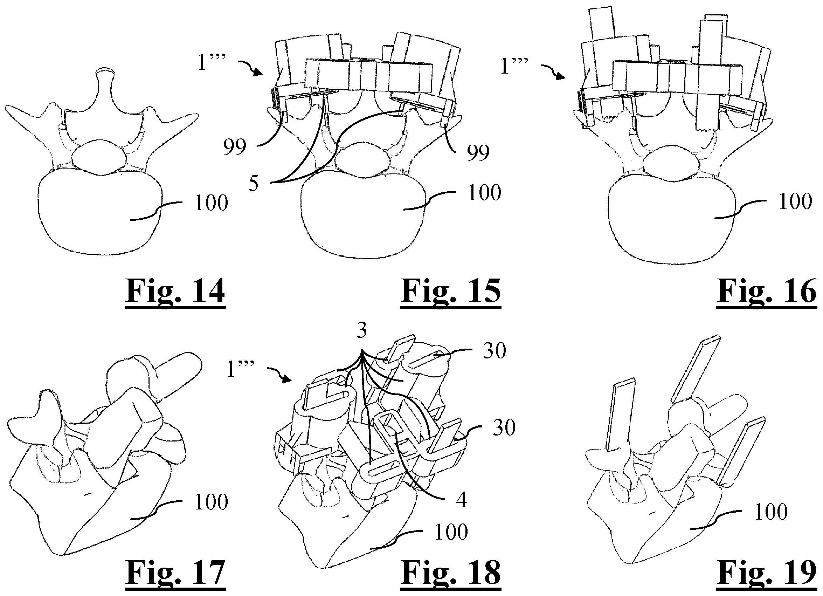

In an example not forming part of the invention, instead of having tubular guiding members 2 meant to be used as drill guides, the operative guide according to the present invention can feature slotted guiding members 3 meant to be used as cutting guides, as depicted in figures 14-19 showing a patient specific operative guide 1"'.

The slotted guiding members 3 are provided with slots 30, meant to guide blades or chisels in order to cut the bone at predefined positions. The slots 30 define potential positions to cut the bone. Potential applications can be PSO (Pedicle substraction osteotomies), laminotomy or facectomies.

The surgical procedure employing a patient specific operative guide 1, 1', 1 ", 1"' comprises a pre-operative planning and an intra-operative procedure.

The pre-operative planning comprises a first step of acquiring CT/MRI scans of the surgical site, a second step of reconstructing a three-dimensional image of the site and a third step of planning the screw placements (or the location of the cuts) on the three-dimensional image by means of computer-aided design tools.

Once the screw axes or the cutting planes have been identified, the steps of designing and producing the patient specific operative guide 1, 1', 1 ", 1"' are performed.

The intra-operative procedure is described below with reference to the patient specific guide 1, 1', 1" according to one of the first three embodiments, i.e. the patient specific drill guide.

The procedure comprises a step of cleaning the vertebra and cutting the ligaments (if necessary) and a subsequent step of coupling the guide to the cleansed vertebra. Note that, prior to the coupling, the correct location and alignment of the guiding members 2 can be checked on a real size three-dimensional model of the vertebra.

After the coupling, two awls are inserted into the tubular guiding members 2. After removal of the awls the surgeon can check the entry points for the pedicle screws. In the next step, the pedicle of the vertebra is opened with a probe or drill inserted in the guiding member 2. The surgeon can use a feeler to help himself in the process. Finally, after removing the probes or drills, the pedicle screws can be inserted via the tubular guiding members 2 by means of a screwdriver.

The window 21 at the bottom end of the tubular guiding members 2 is useful to check the entry points of the pedicle screws, to assess any interference of the instruments with the anatomy of the vertebra and to control if the screw is completely inserted.

In an alternative method, the adapter sleeve is capped on top of the tubular guiding members 2 and two Kirchner wires are inserted into the vertebra instead of directly fixing the pedicle screws. After removal of the operative guide, the Kirchner wires are used to guide the insertion of a cannulated pedicle screw.

Obviously a person skilled in the art, in order to meet specific needs, will readily acknowledge the possibility of changes and variations to the operative guides described above, comprised within the scope of protection as defined by the following claims.

## Claims

1. Patient specific operative guide (1, 1', 1") for use in spinal surgery, comprising at least one couple of guiding members (2) for guiding a surgical operation on a patient's vertebra (100, 100', 100"); and a plurality of contact members (4, 5, 6) designed to match with a corresponding plurality of contact areas on the patient's vertebra (100, 100', 100") in order to define a unique coupling configuration of the patient specific operative guide (1, 1', 1") on the patient's vertebra (100, 100', 100"); said contact members (4, 5, 6) comprising a main contact member (4), designed to couple with a main contact area corresponding to the spinous process (101) of the patient's vertebra (100, 100', 100") in said coupling configuration, and at least one pair of auxiliary contact members (5, 6), designed to abut on auxiliary contact areas, respectively positioned laterally left and laterally right of the spinous process (101), in said coupling configuration, **characterized in that** said at least one couple of guiding members (2 is coupled to the main contact member (4) by a bearing frame (7) comprising a V-shaped bridge (71) having a planar extension and one or more straight transverse bars (70) connecting together upper sections of the guiding members.

2. Patient specific operative guide according to claim 1 **characterized in that** the V-shaped bridge comprises, integrally, the main contact member (4).

3. Patient specific operative guide according to claim 2, **characterized in that** main contact member (4) presents a closed respectively at least partially open profile.

4. Patient specific operative guide according to at least one of the above claims, **characterized in that** said main contact member (4) defines a seat (40) into which the spinous process (101) fits.

5. Patient specific operative guide according to any of the above claims, wherein the contact members (4, 5, 6) are designated to match with five separate contact areas, the auxiliary contact members (5,6) comprising: a pair of first auxiliary contact members (5), matching with first auxiliary contact areas, and a pair of second auxiliary contact members (6), matching with second auxiliary contact areas, respectively positioned laterally left and laterally right of the auxiliary contact areas.

6. Patient specific operative guide, according to any of the previous claims, wherein the guiding members (2) comprise at least a tubular member (2) to be used as drilling guide.

7. Patient specific operative guide, according to claim 6 wherein the diameter of said member (2) is such as to allow the insertion of a pedicle screw and its corresponding fixing/drilling tool, the patient specific guide (1) further comprising an adapter sleeve insertable within the tubular member (2) and featuring a hole for guiding a Kirchner wire.

## Patentansprüche

1. Patientenspezifische Operationsführung (1, 1', 1") zur Verwendung in der Wirbelsäulenchirurgie, umfassend mindestens ein paar Führungselemente (2) für die Führung einer chirurgischen Operation am Wirbel eines Patienten (100, 100', 100"); und eine Vielzahl von Kontaktelementen (4, 5, 6), ausgelegt, um zu einer entsprechenden Vielzahl von Kontaktflächen am Wirbel (100, 100', 100") des Patienten zu passen, um eine einzigartige Kopplungskonfiguration der patientenspezifischen Operationsführung (1, 1', 1") am Wirbel des Patienten (100, 100', 100") zu definieren; wobei die Kontaktelemente (4, 5, 6) ein Hauptkontaktelement (4) umfassen, ausgelegt, um mit einem Hauptkontaktbereich zu koppeln, der dem Dornfortsatz (101) des Patientenwirbels (100, 100', 100") in der Kopplungskonfiguration entspricht und mindestens ein paar von Hilfskontaktelementen (5, 6), ausgelegt, um an Hilfskontaktflächen anzuliegen, die jeweils seitlich links und seitlich rechts des Dornfortsatzes (101) in der Kopplungskonfiguration angeordnet sind, **dadurch gekennzeichnet, dass** das mindestens ein paar Führungselemente (2) mit dem Hauptkontaktteil (4) durch einen Trägerrahmen (7) gekoppelt ist, umfassend ein V-förmige Brücke (71), die eine ebene Verlängerung und eine oder mehrere gerade Querstangen (70) aufweist, die obere Abschnitte der Führungselemente miteinander verbinden.

2. Patientenspezifische Operationsführung nach Anspruch 1, **dadurch gekennzeichnet, dass** die V-förmige Brücke das Hauptkontaktteil (4) integral umfasst.

3. Patientenspezifische Operationsführung nach Anspruch 2, **dadurch gekennzeichnet, dass** das Hauptkontaktelement (4) ein geschlossenes, jeweils mindestens teilweise offenes Profil aufweist.

4. Patientenspezifische Operationsführung nach mindestens einem der vorhergehenden Ansprüche, **dadurch gekennzeichnet, dass** das Hauptkontaktteil (4) einen Sitz (40) definiert, in dem der Dornfortsatz (101) hineinpasst.

5. Patientenspezifische Operationsführung nach einem der vorhergehenden Ansprüche, wobei die Kontaktelemente (4, 5, 6) bestimmt sind, um zu fünf separaten Kontaktflächen zu passen, wobei die Hilfskontaktelemente (5, 6) umfassen: ein paar erste Hilfskontaktelemente (5), die zu den ersten Hilfskontaktflächen passen, und ein paar zweite Hilfskontaktelemente (6), die zu zweiten Hilfskontaktflächen passen, die jeweils seitlich links und seitlich rechts der Hilfskontaktflächen angeordnet sind.

6. Patientenspezifische Operationsführung nach einem der vorhergehenden Ansprüche, wobei die Führungselemente (2) mindestens ein als Bohrführung zu verwendendes rohrförmiges Element (2) aufweisen.

7. Patientenspezifische Operationsführung nach Anspruch 6, wobei der Durchmesser des Elements (2) so beschaffen ist, dass das Einsetzen einer Pedikelschraube und des entsprechenden Befestigungs- /Bohrwerkzeuges ermöglicht wird, wobei die patientenspezifische Führung (1) ferner eine Adapterhülse umfasst, die innerhalb des rohrförmigen Elements (2) einsetzbar ist und ein Loch zur Führung eines Kirschner-Drahtes aufweist.

## Revendications

1. Guide de fonctionnement spécifique au patient (1, 1', 1") destiné à être utilisé en chirurgie vertébrale, comprenant au moins un couple d'éléments de guidage (2) servant à guider une opération chirurgicale sur la vertèbre d'un patient (100, 100', 100") ; et une pluralité d'éléments de contact (4, 5, 6) conçus pour s'adapter à une pluralité correspondante de zones de contact sur la vertèbre du patient (100, 100', 100") afin de définir une unique configuration d'accouplement du guide de fonctionnement spécifique au patient (1, 1', 1") sur la vertèbre du patient (100, 100', 100") ; lesdits éléments de contact (4, 5, 6) comprenant un élément de contact principal (4) conçu pour s'accoupler à une zone de contact principale correspondant à l'apophyse épineuse (101) de la vertèbre du patient (100, 100', 100") dans ladite configuration d'accouplement, et au moins une paire d'éléments de contact auxiliaires (5, 6), conçus pour se mettre en butée sur des zones de contact auxiliaires respectivement positionnées latéralement à gauche et latéralement à droite de l'apophyse épineuse (101) dans ladite configuration d'accouplement, **caractérisé en ce que** ledit au moins un couple d'éléments de guidage (2) est accouplé à l'élément de contact principal (4) par un châssis de support (7) comprenant un pont en « V » (71) comportant une extension planaire et une ou plusieurs barres transversales rectilignes (70)-reliant ensemble des sections supérieures des éléments de guidage.

2. Guide de fonctionnement spécifique au patient selon la revendication 1, **caractérisé en ce que** le pont en « V » comprend, intégralement, l'élément de contact principal (4).

3. Guide de fonctionnement spécifique au patient selon la revendication 2, **caractérisé en ce que** l'élément de contact principal (4) présente respectivement un profil fermé et au moins partiellement ouvert.

4. Guide de fonctionnement spécifique au patient selon au moins une des revendications précédentes, **caractérisé en ce que** ledit élément de contact principal (4) définit un siège (40) dans lequel s'adapte l'apophyse épineuse (101).

5. Guide de fonctionnement spécifique au patient selon l'une quelconque des revendications précédentes, dans lequel les éléments de contact (4, 5, 6) sont désignés pour correspondre à cinq zones de contact séparées, les éléments de contact auxiliaires (5, 6) comprenant : une paire de premiers éléments de contact auxiliaires (5) s'adaptant aux premières zones de contact auxiliaires, et une paire de seconds éléments de contact auxiliaires (6) s'adaptant aux secondes zones de contact auxiliaires, respectivement positionnées latéralement à gauche et latéralement à droite des zones de contact auxiliaires.

6. Guide de fonctionnement spécifique au patient selon l'une quelconque des revendications précédentes, dans lequel les éléments de guidage (2) comprennent au moins un élément tubulaire (2) à utiliser comme guide de perçage.

7. Guide de fonctionnement spécifique au patient selon la revendication 6, dans lequel le diamètre dudit élément (2) est tel qu'il permet l'insertion d'une vis pédiculaire et son outil de fixation/perçage correspondant, le guide spécifique au patient (1) comprenant de plus un manchon de serrage pouvant s'insérer à l'intérieur de l'élément tubulaire (2) et présentant un orifice pour guider une broche de Kirschner.
